# EUROPÄISCHE PATENTSCHRIFT

(11) **EP 1 292 344 B1**
(45) Veröffentlichungstag und Bekanntmachung des Hinweises auf die Patenterteilung: **10.08.2005**
(21) Anmeldenummer: 01953796.8
(22) Anmeldetag: 20.06.2001
(51) Int. Cl.: A61M 5/30

(54) **NADELLOSE INJEKTIONSVORRICHTUNG MIT PYROTECHNISCHEM ANTRIEB**
NEEDLELESS INJECTION DEVICE WITH PYROTECHNIC DRIVE
DISPOSITIF D'INJECTION SANS AIGUILLE A ENTRAINEMENT PYROTECHNIQUE

(30) Priorität: 20.06.2000 DE 10029325
(43) Veröffentlichungstag der Anmeldung: 19.03.2003
(60) Teilanmeldung: 05009528.0
(73) Patentinhaber: Roche Diagnostics GmbH, 68305 Mannheim (DE); F. Hoffmann-La Roche AG, 4070 Basel (CH)
(72) Erfinder: LELL, Peter, 85368 Moosburg (DE)
(74) Vertreter: Jany, Peter, Dr.
(86) Internationale Anmeldenummer: PCT/DE2001/002271
(87) Internationale Veröffentlichungsnummer: WO 2001/097880

(56) Entgegenhaltungen:
- WO-A-01/51109
- US-A- 6 045 534

## Beschreibung

Die Erfindung betrifft eine Treibladungskartusche bzw. eine Kanüle-/Treibladungskartsucheneinheit für eine nadellose Injektionsvorrichtung, mit welcher ein Wirkstoff mittels hohem Druck perkutan oder intramuskulär injiziert werden kann.

Das Grundprinzip, einen Wirkstoff nadellos mittels hohem Druck zu injizieren, ist seit geraumer Zeit bekannt. Aus der US 3,308,818 ist eine Injektionspatrone bekannt, welche ein geschlossenes Gehäuse mit einer düsenartigen Öffnung aufweist. In dem Gehäuse ist eine Treibladung vorgesehen, welche mit einer Anzündvorrichtung aktivierbar ist. Zwischen der Treibladung und der düsenartigen Öffnung ist ein sphärischer Behälter vorgesehen, in welchem der zu injizierende Wirkstoff enthalten ist. Die Injektionspatrone kann in eine revolverartige Injektionsvorrichtung eingesetzt werden, welche einen Auslösemechanismus für die Anzündvorrichtung der Injektionspatrone umfasst. Nach dem Auslösen der Anzündvorrichtung wird die Treibladung aktiviert, wobei durch den im Innenraum der Patrone entstehenden Gasdruck der Wirkstoffbehälter mit hohem Druck beaufschlagt und zusammengequetscht wird. Hierdurch platzt der Behälter im Bereich der düsenartigen Öffnung auf und der zu injizierende Wirkstoff wird mit hohem Druck aus der düsenartigen Öffnung abgegeben.

Aus der WO 98/31409 ist ein nadelloses Injektionssystem bekannt, bei dem ebenfalls in einer einmal verwendbaren Kartusche der zu injizierende Wirkstoff in einem Volumen enthalten ist, welches zumindest größtenteils von einer geschlossenen, ausreichend flexiblen und damit zusammenpressbaren Wandung umgeben ist. Das Volumen weist wiederum eine düsenartige Öffnung auf, welche im Ausgangszustand geschlossen und vor Durchführung der Injektion, beispielsweise durch das Abbrechen eines Verschlusses, geöffnet werden kann. Im rückwärtigen Bereich der Kartusche ist eine Treibladung vorgesehen, sowie eine Anzündvorrichtung zum Aktivieren der Treibladung. Durch das Aktivieren der Treibladung und den hierdurch erzeugten Gasdruck ist der in dem Volumen enthaltene Wirkstoff aus der düsenartigen Öffnung auspressbar.

Nachteilig bei derartigen Injektionspatronen oder -kartuschen ist jedoch, dass der Wirkstoff bereits von vornherein in einem abgeschlossenen Volumen der Patrone bzw. der Kartusche in einer flexiblen Hülle aufgenommen ist und damit für jede Dosierung und jeden bestimmten Wirkstoff eine entsprechende Kartusche oder Patrone hergestellt werden muss. Ein Arzt müsste demzufolge zumindest für Wirkstoffe, bei denen häufig unterschiedliche Dosierungen erforderlich sind, eine große Anzahl unterschiedlicher Kartuschen bevorraten. Des Weiteren kann es erforderlich sein, auch die Treibladung für unterschiedliche Anwendungsfälle, z.B. unterschiedliche Hauttypen, unterschiedliche gewünschte Eindringtiefen des Wirkstoffs etc., zu variieren. Hierdurch wird die Anzahl erforderlicher unterschiedlicher Kartuschen noch erhöht.

Ein weiterer Nachteil derartiger Injektionspatronen besteht darin, dass in jedem Fall sicher gestellt werden muss, dass auch über eine längere Lagerungszeit der Patronen der betreffende Wirkstoff nicht durch das Material der den Wirkstoff einschließenden flexiblen Wandung nachteilig beeinflusst wird. Da eine derartige flexible Wandung jedoch praktisch nur aus Kunststoff, beispielsweise aus PE, hergestellt werden kann, müssen entsprechend hochwertige und auch über längere Zeit hochdichte Materialien verwendet werden. Diese sind jedoch teuer. Des Weiteren muss jeder einzelne Patronentyp ein langwieriges Genehmigungsverfahren durchlaufen, da die Patrone aus solches als Medikament eingestuft wird und somit strengen Zulassungsverfahren unterliegt.

Ein weiterer Nachteil bei derartigen Injektionspatronen ist die Gefahr, dass die den Wirkstoff einschließende flexible Hülle durch den mittels der Treibladung erzeugten Gasdruck oder bei der Gaserzeugung entstehende Partikel zerstört wird. In diesem Fall würde der Wirkstoff mit dem Gas und den erzeugten Partikeln verunreinigt. Dies kann bei den Patienten zu Entzündungen oder allergischen Reaktionen führen.

Zur nadellosen Injektion sind des Weiteren mechanische Vorrichtungen bekannt, welche ein Federsystem aufweisen, das den Kolben einer Injektionskanüle mit einer ausreichend hohen Kraft beaufschlagt und den Kolben so schnell nach vorne bewegt, dass der zu injizierende Wirkstoff mit hohem Druck aus der Austrittsöffnung der Kanüle abgegeben wird. Durch die Verwendung von Einwegkanülen, in denen ein Wirkstoff in üblicher Art erst vor der durchzuführenden Injektion aufgezogen wird, unterliegen derartige Vorrichtungen und Kanülen nicht den strengen Zulassungsvorschriften für Medikamente.

Nachteilig bei rein mechanischen nadellosen Injektionssystemen ist der erforderliche hohe konstruktive Aufwand sowie ein hoher Wartungsaufwand. Zudem ist der in der Kanüle erzeugbare Druck für das Auspressen des Wirkstoffs begrenzt. Unter Umständen müssen daher relativ große Durchmesser für die düsenartige Austrittsöffnung verwendet werden, wodurch die Injektion für den Patienten schmerzhafter ist. Darüber hinaus ist bei einem nicht ausreichend hohen Druck die Eindringtiefe für den Wirkstoff nicht groß genug. Schließlich ermöglichen federgetriebene mechanische Injektionssysteme nur eine verhältnismäßig geringe Beschleunigung des Kolbens der Kanüle, so dass derartige Vorrichtungen für bestimmte Anwendungsfälle, in denen ein sehr rascher Druckanstieg gewünscht ist, nicht verwendbar sind.

Schließlich ist aus der US 5,399,163 ein nadelloses Injektionsverfahren und eine hierzu geeignete Vorrichtung bekannt, bei welcher der Kolben einer den zu injizierenden Wirkstoff enthaltenden Kanüle durch den Gasdruck einer Gaspatrone, die beispielsweise CO2-Gas enthält, beaufschlagbar ist. Diese Vorrichtung weist eine Druckverstärkereinrichtung auf, die durch Federn- und Kolbensysteme gebildet ist. Der damit verbundene konstruktive Aufwand macht ein derartiges System teuer. Zudem muss dieses System, wie alle mechanischen Systeme, häufig gewartet werden. Das System ist infolge seiner Größe und seines Gewichts zudem nur relativ umständlich zu handhaben. Wie alle mechanischen Systeme ist die erreichbare Anstiegsgeschwindigkeit des den Wirkstoff auspressenden Drucks begrenzt.

Ausgehend von diesem Stand der Technik liegt der Erfindung die Aufgabe zu Grunde, eine nadellose Injektionsvorrichtung zu schaffen, die einfach und kostengünstig herstellbar ist, die leicht zu handhaben ist und möglichst universell einsetzbar ist.

Die Erfindung löst diese Aufgabe mit den Merkmalen des Patentanspruchs 1. Die Erfindung geht von der Erkenntnis aus, dass mit einem pyrotechnischen Antrieb, der zur Betätigung des Kolbens einer Kanüle dient, erforderlichenfalls ein extrem steiler Druckanstieg für das Auspressen des Wirkstoffs aus der Kanüle erzeugt werden kann. Durch die Verwendung einer Treibladungskartusche gemäß Anspruch 1 kann diese exakt auf den jeweiligen Einsatzzweck abgestimmt sein. Durch unterschiedliche Treibladungen bzw. unterschiedliche Ausbildungen und Dimensionierungen des Treibspiegels und des Kartuschenvolumens kann der jeweils gewünschte zeitliche Druckverlauf erzeugt werden. Beispielsweise ist es möglich, für die Treibladung eine Kombination aus einem schnellen lebhaften Treibladungspulver und einem langsamen, aber über einen längeren Zeitraum gaserzeugenden Treibladunspulver zu verwenden. Hierdurch kann beispielsweise ein sehr rascher Druckanstieg mit einem hohen Peak und einem sich anschließendem zunächst konstanten und dann langsam abfallenden Druckverlauf erzeugt werden. Unter einer Treibladung soll im Sinne der vorliegenden Erfindung jedes aktivierbare Gas erzeugende Material verstanden werden.

Durch das Vorsehen einer in das Gehäuse der Injektionsvorrichtung einsetzbaren Kanüle, in welche der Wirkstoff erst kurz vor der durchzuführenden Injektion eingebracht wird, ergeben sich an die Materialien der Kanüle geringere Anforderungen.

Zwischen dem zu injizierenden Wirkstoff und der Treibladung bzw. dem durch die Treibladung erzeugten Gas befinden sich sowohl der Treibspiegel als auch der Kolben der Kanüle. Da sowohl der Treibspiege1 als auch der Kolben der Kanüle jeweils mit dem Umfang gegenüber dem jeweiligen Gehäuseteil auch während einer Verschiebebewegung abdichtend ausgebildet sind, ist die Gefahr einer Kontamination des Wirkstoffs durch das Treibladungsgas bzw. Partikel der Treibladung praktisch ausgeschlossen. Der Treibspiegel kann im jeweiligen Gehäuse in seiner Ausgangsposition rastend gehalten sein. Des Weiteren können auch Mittel zur rastenden Halterung des Treibspiegels in einer Endposition vorgesehen sein.

Damit wird verhindert, dass der Treibspiegel bei einer Bewegung der Vorrichtung aus seiner Ausgangsposition herausbewegt wird, ohne dass der Treibspiegel mit dem Gasdruck der Treibladung beaufschlagt wird. Die rastende Halterung des Treibspiegels in der Endposition gewährleistet, dass beim Austauschen der Kartusche der Treibspiegel nicht aus der Kartusche herausfällt oder in der Injektionsvorrichtung verbleibt. Zudem bleibt bei einer derart ausgebildeten Kartusche dauernd sichtbar, dass die Kartusche bereits benutzt wurde, da der Treibspiegel nicht unabsichtlich wieder in seine Ausgangsposition bewegbar ist.

In der bevorzugten Ausführungsform kann der Treibspiegel auf seiner mit dem Gasdruck beaufschlagbaren Stirnseite im äußeren Bereich einer ringförmige Ausnehmung aufweisen, die so beschaffen ist, dass die zwischen dem Außenumfang des Treibspiegels und der ringförmigen Ausnehmung ausgebildete lidartige Wandung bei Beaufschlagung mit dem Gasdruck unter vorzugsweise elastischer Verformung gegen die Innenwandung des jeweiligen Gehäuses gepresst wird, um eine Dichtwirkung zu erzeugen. Durch diese einfache Maßnahme kann auf das Vorsehen eines O-Rings am Außenumfang des Treibspiegels verzichtet werden. Die Herstellung wird damit vereinfacht und verbilligt.

Nach einer Ausführungsform kann der Treibspiegel in einem Gehäuseteil der Injektionsvorrichtung vorgesehen sein, wobei die Treibladungskartusche als separates Teil ausgebildet und in das Gehäuseteil der Injektionsvorrichtung einsetzbar ist. Der Treibspiegel kann bei dieser Ausführungsform gegen die Rückstellkraft eines federnden Elements bewegbar sein. Hierdurch wird eine mehrfache Verwendbarkeit des Treibspiegels erreicht. Die Treibladungskartusche weist in diesem Fall keinen eigenen Treibspiegel auf, sondern ist als reine Gas erzeugende Kartusche ausgebildet. Allerdings ergeben sich bei einer derartigen Ausführungsform in dem Gehäuseteil, in dem der Treibspiegel verschiebbar gehalten ist, Schmauchspuren, so dass diese Gehäuseteil nur in begrenztem Rahmen mehrfach verwendbar ist. Das Gehäuseteil müsste häufig gereinigt und gegebenenfalls ausgetauscht werden.

Das Gehäuseteil kann in diesem Fall zusammen mit dem Treibspiegel als kartuschenartiges Austauschteil ausgebildet sein.

In einer anderen Ausführungsform ist der Treibspiegel im Gehäuse einer Kanüle vorgesehen und die Kartusche als separates Teil ausgebildet und in das Kanülengehäuse einsetzbar.

In gleicher Weise kann der Treibspiegel wiederum im Kanülengehäuse vorgesehen sein, wobei die Kartusche als separates Teil ausgebildet und an der rückwärtigen Seite der Kanüle in das Gehäuse der Injektionsvorrichtung einsetzbar sein.

Auch in diesen Fällen kann die Kartusche als reine Gas erzeugende Kartusche ohne eigenen Treibspiegel ausgebildet sein.

In einer weiteren Ausführungsform können die Kanüle und die Kartusche als ein einziges Teil ausgebildet sein, wobei der Kolben und der Treibspiegel im gemeinsamen Kanülen-/Kartuschengehäuse vorgesehen sind. Diese Ausführungsform bietet sich beispielsweise für solche Einsatzzwecke an, in denen sehr häufig dieselbe Kanülengröße mit derselben Treibladung verwendbar ist.

In allen Fällen, in denen der Treibspiegel im Kanülengehäuse oder im gemeinsamen Kanüle-/Kartuschengehäuse vorgesehen ist, kann der Treibspiegel einstückig mit dem Kolben ausgebildet oder kraftschlüssig mit diesem gekoppelt sein.

Dabei kann der axiale Abstand zwischen den Dichtungsmitteln des Kolbens und den Dichtungsmitteln des Treibspiegels größer sein, als der Bewegungsweg des Treibspiegels zwischen seiner Ausgangsposition und seiner Endposition. Hierdurch ergibt sich der Vorteil, dass nicht ein und derselbe Teilbereich der Innenwandung des Gehäuses sowohl mit den Dichtungsmitteln des Treibspiegels als auch mit den Dichtungsmitteln des Kolbens zusammenwirken muss, um eine abdichtende Wirkung zu erzielen. Hierdurch ergibt sich eine verbesserte Betriebssicherheit im Hinblick auf eine Kontamination des Wirkstoffs durch das Treibgas bzw. Partikel des Treibgases.

In einer anderen Ausführungsform können die Kanüle und die Treibladungskartusche jeweils als separates Teil ausgebildet und in die Injektionsvorrichtung einsetzbar sein. Der Treibspiegel ist hierbei in der Treibladungskartusche vorgesehen und beaufschlagt vorzugsweise bereits nach dem Einsetzen in die Injektionsvorrichtung den Kolben der Kanüle. Bei dieser Ausführungsform wird der Treibspiegel lediglich innerhalb des Kartuschengehäuses bewegt und ist gegenüber diesem abgedichtet. Außerhalb des Kartuschengehäuses werden daher kaum Schmauchspuren erzeugt. (Eine derartige Treibladungskartusche kann auch für andere Einsatzzwecke als mechanisch wirkende pyrotechnische Antriebsvorrichtungen verwendet werden.)

Die Kanüle (ob als separates Teil oder als kombinierte Kanülen-/Kartuscheneinheit) kann nach dem Einsetzen in die Injektionsvorrichtung im Wesentlichen dem gesamten Außenumfang durch das Gehäuse der Injektionsvorrichtung abgestützt sein. Hierdurch kann die Wandstärke der Kanüle geringer ausgebildet sein, da nicht der gesamte Druck von der Kanülenwandung aufgefangen werden muss.

Erfindungsgemäß weist die Treibladungskartusche mit oder ohne Treibspiegel oder eine Kanüle-/Treibladungskartsucheneinheit ein Co-Volumen auf, welches mit dem Volumen des Gehäuses, in welchem das Gas erzeugbar ist, in der Ausgangsposition des Treibspiegels verbunden oder durch Bewegen des Treibspiegels aus seiner Ausgangsposition heraus verbindbar ist.

Durch das Vorsehen des Co-Volumens kann der Druckanstieg und auch der folgende zeitliche Verlauf der auf den Treibspiegel ausgeübten Druckkraft beeinflusst werden.

Das Co-Volumen kann als Ringraum ausgebildet sein, welcher sich um das Volumen erstreckt, in welchem das Gas erzeugbar ist.

Der Ringraum kann durch eine stirnseitige Wandung begrenzt sein, in welcher ein oder mehrere Durchbrüche vorgesehen sind, die in der Ausgangsposition durch den Treibspiegel im Wesentlichen abgedichtet sind. In der stirnseitigen Wandung ist zumindest ein weiterer, vorzugsweise zentrischer Durchbruch vorgesehen, durch welchen das durch die Treibladung erzeugte Gas den Treibspiegel beaufschlagt.

Das Co-Volumen des Ringraums kann durch ein an der rückwärtigen Stirnseite des Ringraums eingreifendes ringförmiges Element einstellbar sein. Das ringförmige Element kann beispielsweise durch einen Verstellmechanismus verschiebbar sein oder es können für unterschiedliche Anwendungszwecke unterschiedliche Ringelemente verwendet werden, die in den Ringraum eingesetzt werden.

Die Anzündvorrichtung der erfindungsgemäßen Treibladungskartusche oder der erfindungsgemäßen Kanülen-/Treibladungskartuscheneinheit ist vorzugsweise elektrisch ansteuerbar ausgebildet, wobei zwei Anschlusskontakte der Anzündvorrichtung an der rückwärtigen Stirnseite des jeweiligen Gehäuses so herausgeführt sind, dass deren Kontaktanschlussflächen innerhalb zweier gedachter, sich nicht überlappender, konzentrischer Ringbereiche oder innerhalb eines gedachten zentrischen Kreises und eines gedachten konzentrischen Ringbereichs liegen.

Die Anzündeinrichtung der nadellosen Injektionsvorrichtung für eine derartige Treibladungskartusche ohne Kanülen-/Treibladungskartsucheneinheit kann zwei ringförmige, schneidenartig ausgebildete Kontakte oder einen zentrischen Kontakt und einen ringförmigen, schneidenartig ausgebildeten Kontakt aufweisen. Durch diese Ausbildung der Anschlusskontakte der Anzündvorrichtung der Treibladungskartusche bzw. Kanülen-/Treibladunskartuscheneinheit und die Ausbildung der Kontakte der Anzündeinrichtung ergibt sich eine sichere elektrische Verbindung zwischen den betreffenden Kontakten nach dem Einsetzen der Treibladungskartusche bzw. Kanülen-/Treibladungskartuscheneinheit in die Injektionsvorrichtung, ohne die Kartusche ausgerichtet einsetzen zu müssen.

Die Anzündeinrichtung umfasst vorzugsweise eine gegen die Rückstellkraft eines federnden Elements verschiebbare Batterie, wobei elektrische Verbindungsleitungen vorgesehen sind, mittels welcher die Batteriepole bei einem Verschieben der Batterie um wenigstens einen vorbestimmten Weg mit den Kontakten der Anzündeinrichtung verbindbar sind.

Die Batterie kann sich mit einem Batteriepol gegen eine elektrisch leitende flexible Membran abstützen, welche Teil der betreffenden elektrischen Verbindungsleitung ist. Die Membran ist dabei so ausgebildet, dass sie bei Beaufschlagung mit einer Druckkraft das Verschieben der Batterie um einen ausreichenden Weg ermöglicht.

Diese Ausführungsform einer Anzündeinrichtung ist mit geringem Aufwand und damit kostengünstig realisierbar. Durch den mechanisch sehr einfachen Aufbau ergibt sich eine hohe Betriebssicherheit. Sie kann, ebenso wie die spezielle Ausbildung der Kontakte der Anzündvorrichtung, auch für jede andere Vorrichtung eingesetzt werden.

Die Erfindung wird nachfolgend anhand in der Zeichnung dargestellter Ausführungsbeispiele näher erläutert. In der Zeichnung zeigen
- Fig. 1: einen Längsschnitt durch eine Injektionsvorrichtung mit einer separaten Kanüle und einer separaten Treibladungskartusche, wobei der Treibspiegel in der Treibladungskartusche vorgesehen ist;
- Fig. 2: einen Längsschnitt durch eine Injektionsvorrichtung analog Fig. 1 mit einer in die Injektionsvorrichtung einschraubbaren Kanüle;
- Fig. 3: einen Längsschnitt durch einen Injektionsvorrichtung analog Fig. 1 mit einer mittels eines Bajonettverschlusses in die Injektionsvorrichtung einsetzbaren Kanüle;
- Fig. 4: einen Längsschnitt durch eine Injektionsvorrichtung mit einer kombinierten Kanülen-/Treibladungskartuscheneinheit;
- Fig. 5: einen Längsschnitt durch eine Injektionsvorrichtung mit einer Kanüle, in welcher ein mit dem Kolben gekoppelter Treibspiegel vorgesehen ist, und mit einer separaten Treibladungskartusche ohne Treibspiegel;
- Fig. 6: einen Längsschnitt durch eine Injektionsvorichtung mit einem mehrfach verwendbaren Gehäuseteil, in welchem ein Treibspiegel vorgesehen ist und in welches eine Treibladungskartusche einsetzbar ist.
- Fig. 7: einen Längsschnitt durch eine Injektionsvorrichtung analog Fig. 6, jedoch mit einer abgewandelten Treibladungskartusche
- Fig. 8: einen Längsschnitt durch den vorderen Bereich einer Injektionsvorrichtung mit jeweils separat ausgebildeter Kanüle und erfindungsgemäßer Treibladungskartusche, wobei in der Treibladungskartusche ein Co-Volumen ausgebildet ist.
- Fig. 9: einen Längsschnitt durch den vorderen Bereich einer Injektionsvorrichtung analog Fig. 8, mit einer abgewandelten Treibladungskartusche;
- Fig. 10: eine Ausführungsform einer rein Gas erzeugenden Treibladungskartusche (Fig. 10a) und ein Diagramm mit möglichen zeitlichen Verläufen des durch die Treibladungskartusche erzeugbaren Drucks (Fig. 10b);
- Fig. 11: Darstellungen analog Fig. 10, jedoch mit einer anderen Ausführungsform einer rein Gas erzeugenden Treibladungskartusche und
- Fig. 12: eine schematische Draufsicht auf die Rückseite erfindungsgemäßer Treibladungskartuschen oder Kanülen-/Treibladungskartuscheneinheiten.

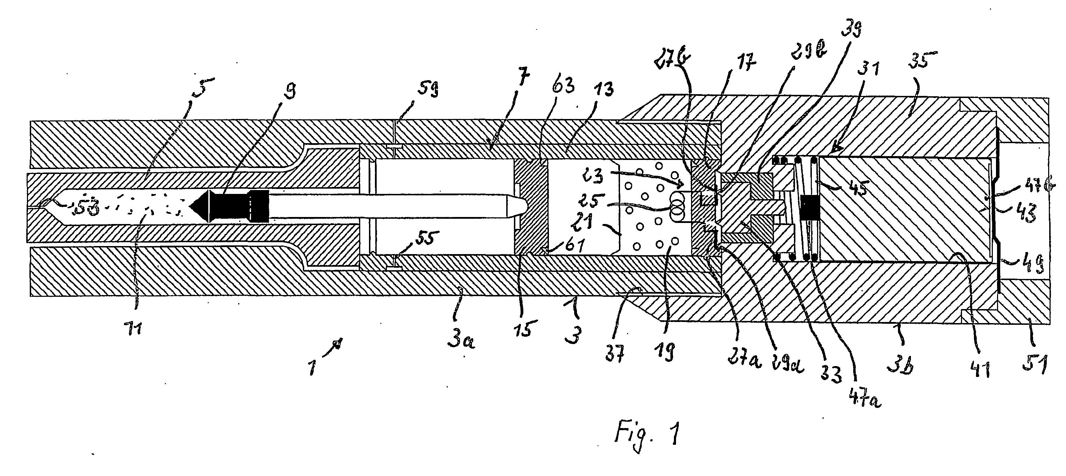

Fig. 1 zeigt eine Injektionsvorrichtung 1 mit einem Gehäuse 3, welches ein vorderes Gehäuseteil 3a und ein rückwärtiges Gehäuseteil 3b aufweist. Das vordere Gehäuseteil 3a dient zur Aufnahme einer Kanüle 5 und einer Treibladungskartusche 7. Bei der in Fig. 1 dargestellten Ausführungsform ist der Aufnahmebereich des vorderen Gehäuseteils 3a für die Kanüle 5 und die Treibladungskartusche 7 so gestaltet, dass die Kanüle und die Treibladungskartusche vom rückwärtigen Ende des Gehäuseteils einschiebbar sind.

Selbstverständlich kann das vordere Gehäuseteil 3a für das Einsetzen von Kanüle und Treibladungskartusche auch aufklappbar und nach dem Verschließen verriegelbar ausgebildet sein.

Bei der dargestellten Ausführungsform ist die Kanüle 5 im Wesentlichen ganz im Gehäuseteil 3 a aufgenommen und von dessen Innenwandung abgestützt. Die Außenkontur der Kanüle 5 und die Kontur der Innenwandung des vorderen Bereichs des vorderen Gehäusebereichs 3a sind hierzu komplementär ausgebildet. Durch diese Maßnahme wird erreicht, dass die Kanüle schwächer dimensionierte Außenwandungen aufweisen kann, da der Druck, den der Kolben 9 der Kanüle 5 auf den im vorderen Bereich der Kanüle aufgenommenen Wirkstoff ausübt durch die Wandung des vorderen Gehäuseteils 3a aufgenommen wird. Trotz dünner Kanülenwandungen und eines hohen Drucks im vorderen Bereich der Kanüle können sich die Wandungen der Kanüle nicht in radialer Richtung nach außen bewegen. Die abdichtende Wirkung zwischen der Außenwandung des Kolbens und der Innenwandung der Kanüle ist somit gewährleistet.

Im rückwärtigen Bereich des vorderen Gehäuseteils 3a ist die Treibladungskartusche 7 aufgenommen, welche eine vorzugsweise zylindrische Wandung 13 in Form eines Rohrabschnitts aufweist. In der zylindrischen Wandung 13 ist ein Treibspiegel 15 vorgesehen. Im rückwärtigen Bereich der Treibladungskartusche 7 ist diese durch ein scheibenartiges Verschlusselement 17 dicht verschlossen. Sowohl die zylindrische Wandung 13 als auch der Treibspiegel 15 und das Verschlusselement 17 können aus Kunststoff bestehen. Das Verschlusselement 17 kann rastend mit der zylindrischen Wandung 13 verbunden sein. Vorzugsweise weist hierzu das Verschlusselement 17 an seinem Außenumfang eine ringförmige Erhebung auf, welche in eine ringförmige Nut in der Innenwandung der zylindrischen Wandung 13 eingreift. Selbstverständlich kann das Verschlusselement 17 auch auf andere Weise mit der Wandung 13 verbunden sein, z.B. durch Verquetschen, Verschweißen oder dergleichen.

Im rückwärtigen Bereich der Treibladungskartusche 7, vorzugsweise unmittelbar vor dem Verschlusselement 17, ist eine Treibladung 19 vorgesehen. Diese besteht vorzugsweise aus einem pyrotechnischen Material und ist mittels eines Rückhalteelement 21, das beispielsweise aus Pappe bestehen kann, im rückwärtigen Bereich der Treibladungskartusche 7 gehalten.

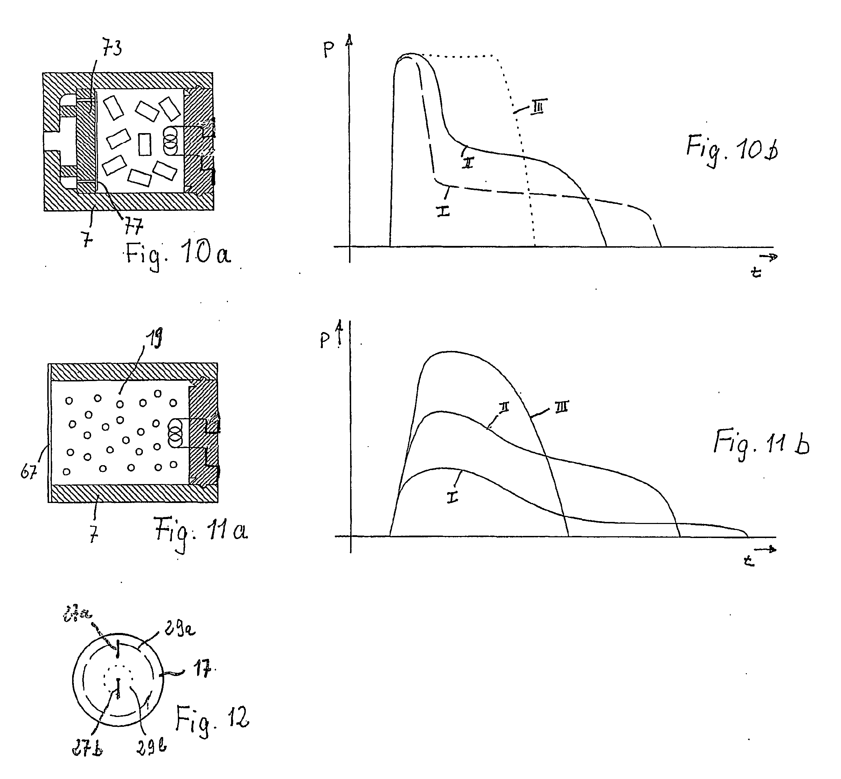

Im rückwärtigen Bereich der Treibladungskartusche ist eine Anzündvorrichtung 23 vorgesehen, die z.B. aus einer Glühwendel 25 bestehen kann, die in den Bereich mit der Treibladung 19 hineinreicht. Die Anschlusskontakte 27a, 27b der Anzündvorrichtung 23 sind an der rückwärtigen Stirnseite des Verschlusselements 17 herausgeführt. Wie in Fig. 12, welche eine schematische Draufsicht auf das Verschlusselement 17 zeigt, ersichtlich, können die Anschlusskontakte 27a, 27b der Anzündvorrichtung 23 mit ihren Kontaktanschlussflächen im Wesentlichen in radialer Richtung auf der Stirnseite des Verschlusselements 17 verlaufen. Der Anschlusskontakt 27b, der im Wesentlichen zentrisch an der Stirnseite des Verschlusselement 17 herausgeführt ist, verläuft ausgehend von seinem zentrischen Durchstoßpunkt radial nach außen. Das radial äußere Ende des Anschlusskontakts 27b endet in radialer Richtung vor dem gedachten Kreis, der durch den Durchstoßpunkt des Anschlusskontakts 27a verläuft. Auf diese Weise ist es möglich, die Anschlusskontakte 27a, 27b mittels ringförmig verlaufender Kontakte 29a, 29b einer Anzündeinrichtung 31 zu kontaktieren, die vorzugsweise als ringförmig verlaufende Schneidkontakte ausgebildet sind. In Fig.12 sind die Linien der Schneidkontatkte 29a, 29b strichliert angedeutet.

Die Anzündeinrichtung 31, die im rückwärtigen Gehäuseteil 3b vorgesehen ist, umfasst ein erstes Kontaktelement 33, welches den ringförmigen Schneidkontakt 29b trägt. Das Kontaktelement 33 ist in einem vorderen Teil 35 des rückwärtigen Gehäuseteils 3b derart gehalten, dass nach dem Einsetzen des rückwärtigen Bereichs des vorderen Gehäuseteils 3a in eine Aufnahmeausnehmung 37 im vorderen Bereich des vorderen Teils 35 der Schneidkontakt 29b des Kontaktelements 33 einen elektrischen Kontakt mit dem Anschlusskontakt 27b der Anzündvorrichtung 23 gewährleistet ist. Das Verbinden des vorderen Teils 35 des rückwärtigen Gehäuseteils 3b und des vorderen Gehäuseteils 3a kann beispielsweise mittels einer Schraubverbindung erfolgen. Das vordere Teil 35 des rückwärtigen Gehäuseteils 3b besteht vorzugsweise aus Metall, so dass in der Aufnahmeausnehmung 37 der Kontakt 29a als ringförmige Erhebung an der bodenseitigen Wandung der Aufnahmeausnehmung 37 ausgebildet sein kann. Zur elektrischen Isolation der Kontakte 29a, 29b ist das Kontaktelement 33 mittels eines Isolierteils 39 im vorderen Teil 35 des rückwärtigen Gehäuseteils 3b gehalten. In einer Aufnahmeausnehmung 41 im rückwärtigen Bereich des Teils 35 ist eine Batterie 43 aufgenommen. Die Batterie ist in der axial verlaufenden Aufnahmeausnehmung 41 axial gegen die Kraft einer Feder 45 in Richtung auf das Kontaktelement 33 verschiebbar. Im Ausgangszustand gewährleistet die Feder 45, dass der elektrische Kontakt zwischen dem Kontaktelement 33 und dem vorderen Batteriepol 47a unterbrochen ist.

Der rückwärtige Batteriepol 47b wird von einer elektrisch leitenden flexiblen oder elastischen Membran 49 beaufschlagt, wobei die Membran 49 einen dauernden oder zumindest bei Andrücken der Membran gegen den rückwärtigen Batteriepol 47b bestehenden elektrischen Kontakt zwischen dem Batteriepol 47b und dem Teil 35 herstellt. Die Membran ist vorzugsweise mittels eines hinteren Teils 51 des rückwärtigen Gehäuseteils 3b mit dem Teil 35 verbunden, wobei die Membran 49 zwischen einander gegenüberstehenden Stirnseiten der Teile 35 und 51 eingeklemmt sein kann.

Wird auf die Membran 49 eine Druckkraft ausgeübt, die ausreicht, um die Batterie 43 gegen die Kraft der Feder 45 so weit axial zu verschieben, dass der vordere Batteriepol 47a das Kontaktelement 33 kontaktiert, so wird die Anzündvorrichtung 23 aktiviert. Im in Fig. 1 dargestellten Fall wird die Treibladung 19 mittels der Glühwendel 25, die infolge des Stromflusses erhitzt wird, angezündet.

Das erzeugte Treibgas wirkt auf den Treibspiegel 15, der den Kolben 9 beaufschlagt. Hierdurch wird der Wirkstoff 11 mit ausreichend hohem Druck in Form eines scharfen Strahls aus der düsenartigen Austrittsöffnung 53 ausgepresst.

Wie in Fig. 1 dargestellt, kann der Treibspiegel 15 in der in Fig. 1 dargestellten Ausgangsstellung mit der zylindrischen Wandung 13 der Treibladungskartusche 7 verrastet sein. Ein unbeabsichtigtes Verschieben des Treibspiegels aus seiner Ausgangsposition wird hierdurch verhindert. Das Verrasten erfolgt vorzugsweise mittels einer an der Innenwandung der Wandung 13 der Kartusche 7 ausgebildeten Erhebung, welche in eine ringförmige Nut im Außenumfang des Treibspiegels eingreift. Hierdurch ist es bei der Montage der Treibladungskartusche 7 möglich, zunächst den Treibspiegel 15 vom rückwärtigen Ende der Treibladungskartusche her einzuführen und einen derartigen Druck auf den Treibspiegel auszuüben, bis dieser in der Ausgangsstellung verrastet. Die in umgekehrter Weise ausgebildete Verrastung zwischen dem Verschlusselement 17 und der Wandung 13 ermöglicht ein einfaches Einschieben des Treibspiegels 15 bei der Montage.

In der Endposition des Treibspiegels 15 im vorderen Bereich der Treibladungskartusche 7 kann an der Innenwandung der Wandung 13 ebenfalls eine Rasterhebung ausgebildet sein. Auf diese Weise wird der Treibspiegel sicher in der Endposition fixiert und kann beim Entnehmen der Treibladungskartusche 7 aus der Injektionseinrichtung 1 nicht herausfallen oder in der Injektionsvorrichtung verbleiben. Die Kanüle dient bei der in Fig. 1 dargestellten Ausführungsform mit ihrem rückwärtigen Ende gleichzeitig als Anschlag für den Treibspiegel 15 in seiner Endposition. Selbstverständlich kann jedoch auch innerhalb der Wandung 13 der Treibladungskartusche 7 ein entsprechender Anschlag vorgesehen sein.

Die Treibladungskartusche 7 weist in ihrem vorderen Bereich eine oder mehrere Luftauslassöffnungen 55 auf, die in eine ringförmige Nut 57 im Außenumfang der Treibladungskartusche 7 münden. Im vorderen Gehäuseteil 3a sind ebenfalls eine oder mehrere Luftauslassöffnungen 59 vorgesehen, die mit der Ringnut 57 kommunizieren. Hierdurch kann die vor dem Treibspiegel befindliche Luft bei dessen Vorwärtsbewegung aus der Injektionsvorrichtung austreten, so dass ein ungewolltes Abbremsen des Treibspiegels vermieden wird.

Der Treibspiegel kann in seiner rückwärtigen Stirnseite eine umlaufende Ausnehmung 61 aufweisen, die so ausgebildet ist, dass am rückwärtigen äußeren Umfangsbereich des Treibspiegels 15 ein lidartiger ringförmiger Teilbereich 63 entsteht. Bei einer Beaufschlagung des Treibspiegels mit dem Treibladungsgas wird der lidartige Teilbereich 63 gegen die Innenwandung der Wandung 13 der Treibladungskartusche 7 gepresst, wodurch eine abdichtende Wirkung entsteht.

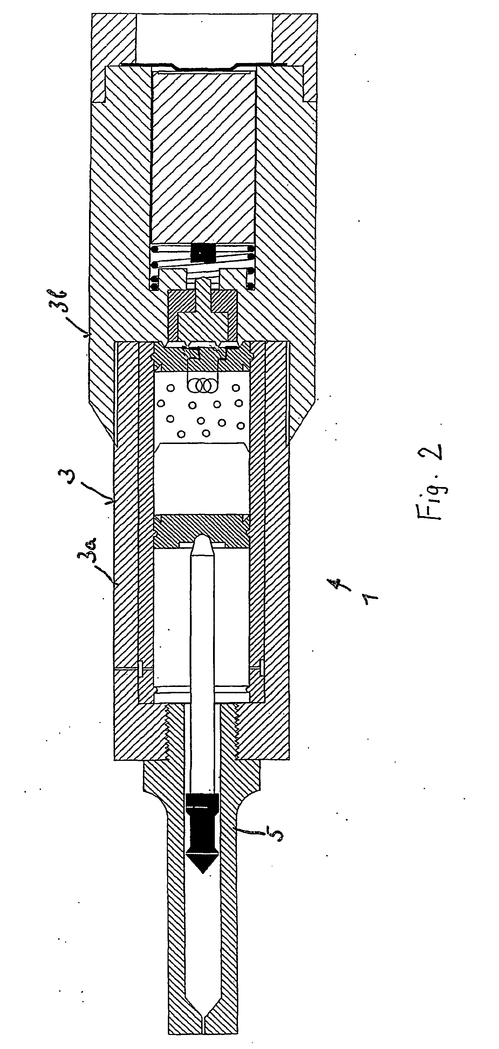

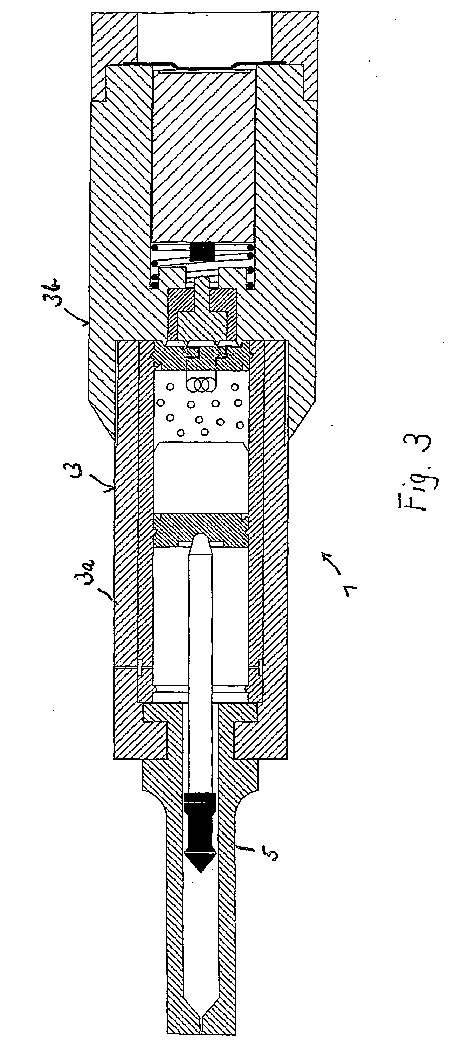

Die in Fig. 2 dargestellte Ausführungsform einer Injektionsvorrichtung 1 unterscheidet sich von der Ausführungsform nach Fig. 1 dadurch, dass die Kanüle 5 in eine Ausnehmung in der Stirnseite des vorderen Gehäuseteils 3a einschraubbar ausgebildet ist. Dementsprechend muss bei dieser Ausführungsform die Wandung der Kartusche stärker dimensioniert sein. Bei der in Fig. 3 dargestellten Ausführungsform ist die Kanüle 5 mittels eines Bajonettverschlusses mit dem Gehäuseteil 3a verbindbar. Der rückwärtige Bereich der Kanüle 5 und die Aufnahmeöffnung in der Stirnseite des Gehäuseteils 3a sind hierzu entsprechend ausgebildet. Auch bei dieser Ausführungsform muss die Wandung der Kanüle 5 stärker dimensioniert sein als bei der Ausführungsform gemäß Fig. 1.

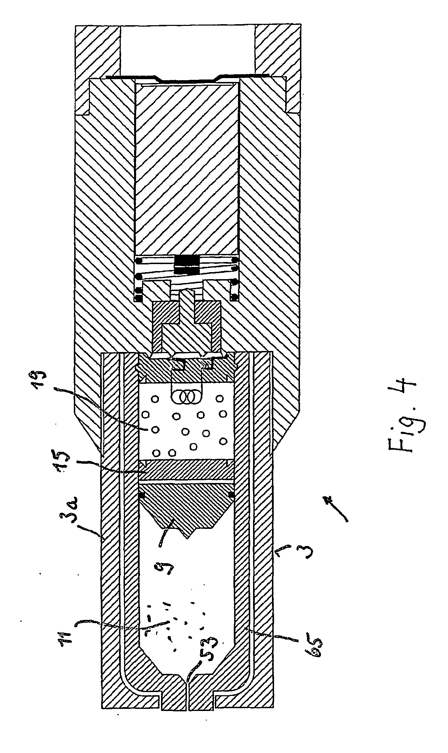

Fig. 4 zeigt eine Ausführungsform einer Injektionsvorrichtung 1, bei der die Kanüle und die Treibladungskartusche als integrierter Kanülen-/Treibladungskartuscheneinheit 65 ausgebildet sind. Im vorderen Kanülenteil der Kanülen-/Treibladungskartuscheneinheit 65 ist ein Kolben 9 vorgesehen, der zum Auspressen des vor dem Kolben befindlichen Wirkstoffs 11 aus der Austrittsöffnung 53 dient. Im rückwärtigen Treibladungskartuschenbereich der Kanülen-/Treibladungskartuscheneinheit 65 ist ein Treibspiegel 15 vorgesehen, der bei einem Anzünden der Treibladung 19 den Kolben 9 beaufschlagt und diesen nach vorne drückt.

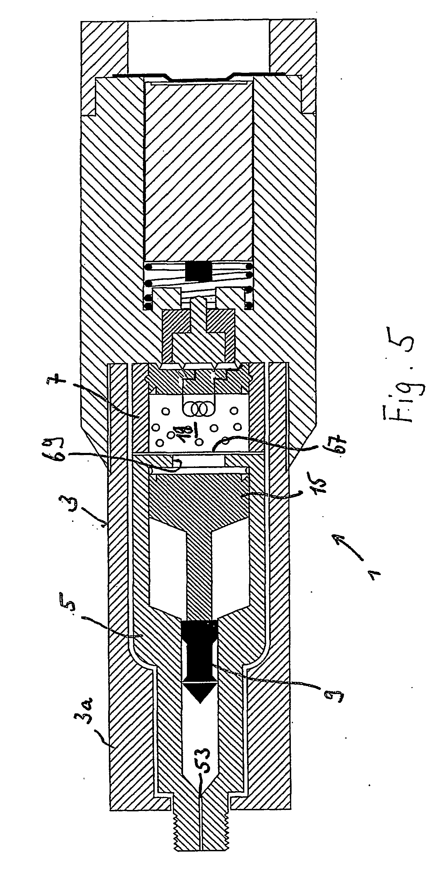

Bei der in Fig. 5 dargestellten Ausführungsform einer Injektionsvorrichtung 1 ist eine Kanüle 5 verwendet, in welcher ein Kolben 9 und ein damit starr verbundener Treibspiegel 15 vorgesehen ist. Ebenso wie bei den Ausführungsformen nach den Fig. 1 und 4 ist die Kanüle 5 im Wesentlichen ganz im Gehäuseteil 3a aufgenommen. Im rückwärtigen Bereich in unmittelbarer Nachbarschaft zur Kanüle 5 ist eine Treibladungskartusche 7 im vorderen Gehäuseteil 3a aufgenommen, welche als reine gaserzeugende Kartusche ausgebildet ist. In dieser Ausführungsform der Kartusche 7 ist kein Treibladungsspiegel enthalten. Die Treibladungskartusche 7 in Fig. 5 ist an ihrer vorderen Stirnseite mit einer Membran 67 verschlossen. Die Membran 67 hält zum Einen die Treibladung 19 im Innenraum der Treibladungskartusche 7 und zum Anderen erfüllt sie die Wirkung einer Verdämmung. Wird die Treibladung 19 angezündet, so dichtet die Membran 67 den Innenraum der Treibladungskartusche 7 so lange ab, bis der Gasdruck einen vorbestimmbaren Schwellwert überschreitet und die Membran 67 bricht oder platzt. Der Schwellendruck kann u.a. bestimmt werden durch die Dicke der Membran 67 und gegebenenfalls eine zusätzliche Abstützung der Membran.

Wie in Fig. 5 dargestellt, kann die Kanüle 5 an ihrem rückwärtigen Ende einen Durchbruch 69 aufweisen, so dass die verbleibenden Bereiche der rückwärtigen stirnseitigen Wandung der Kanüle 5 die Membran 67 abstützen. Abhängig vom Durchmesser des Durchbruchs 69 wird die Membran 67 bei einem höheren Schwellendruck (bei kleineren Durchmessern des Durchbruchs 69) oder niedrigeren Schwellendrücken (bei größeren Durchmessern des Durchbruchs 69) aufplatzen.

Die Ausbildung der Kanüle 5 gemäß Fig. 5 bietet den Vorteil, dass der Kolben und der Treibspiegel jeweils in eigenen Bereichen des Gehäuses der Kanüle 5 geführt wird. Dies verbessert die Betriebssicherheit, da bei einer Beschädigung der Innenwandung der Kanüle 5 nicht die Dichtungswirkung beider Elemente beeinträchtigt wird.

Selbstverständlich kann an Stelle einer starren Verbindung des Kolbens 9 und des Treibspiegels 15 der Kolben kraftschlüssig mit dem Treibspiegel 15 verbunden sein. Hierdurch ergibt sich der Vorteil, dass der Treibspiegel 15 in seiner Ausgangsposition verbleiben kann, und der Kolben 9 in seiner Ausgangsposition im vorderen Bereich der Kanüle vorgesehen sein kann. Erst bei einem Befüllen der Kanüle mit dem Wirkstoff durch die Austrittsöffnung 53 wird der Kolben 9 maximal so weit zurückgeschoben, bis er mit seiner Rückseite die Vorderseite des Treibspiegels 15 beaufschlagt.

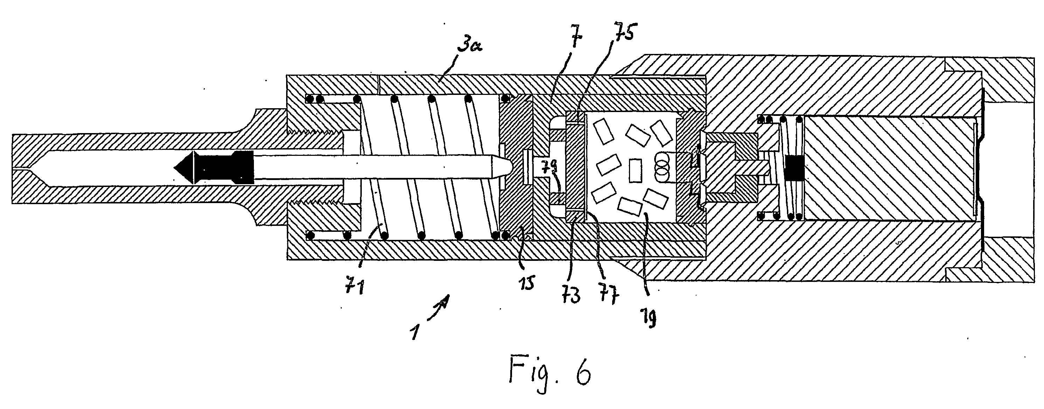

Die in Fig. 6 dargestellte Injektionsvorrichtung 1 umfasst ein vorderes Gehäuseteil 3a, in welchem ein Treibspiegel 15 gegen die Kraft einer Feder 71 verschiebbar ist. Im rückwärtigen Bereich des Gehäuseteils 3a ist wiederum eine rein gaserzeugende Treibladungskartusche 7 aufgenommen.

Das Gehäuseteil 3a ist bei dieser Ausführungsform einer Injektionsvorrichtung als Austauschkartusche gestaltet, da bei einem Anzünden der Treibladung 19 im Innenraum des Gehäuseteils 3a Schmauchspuren entstehen. Das Gehäuseteil 3a und der Treibspiegel 15 sind in diesem Fall zwar mehrfach verwendbar, jedoch ist ein häufiges Reinigen des Innenraums 3a erforderlich und die Kartusche in Form des Gehäuseteils 3a muss nach einer bestimmten maximalen Zahl von Verwendungen ausgetauscht werden.

Die Treibladungskartusche 7 unterscheidet sich von der ebenfalls rein gaserzeugenden Kartusche der Vorrichtung in Fig. 5 dadurch, dass im vorderen Bereich der Treibladungskartusche eine Wandung 73 vorgesehen ist, die mehrere Durchbrüche 75 geringen Querschnitts aufweist. An der Innenseite der Wandung 73 sind die Durchbrüche 75 mittels einer Membran 77 verschlossen. An ihrer Vorderseite weist die Wandung 73 mehrere Stützfüße 79 auf, die sich gegen eine stirnseitige Wandung der Treibladungskartusche 7 abstützen. Die Stützfüße 79 gewährleisten zum Einen, dass die Durchbrüche 75 nicht durch die stirnseitige Wandung der Treibladungskartusche 7 dicht verschlossen werden (in Verbindung mit der stirnseitigen Wandung der Kartusche 7), dass die Wandung 73 nicht aus der Treibladungskartusche 7 herausgedrückt wird oder bricht.

Infolge des geringen Querschnitts der Durchbrüche 75 kann eine nach dem Anzünden relativ langsam reagierende (d.h. einen langsamen Druckanstieg erzeugende) Treibladung verwendet werden. Durch die Verdämmung mittels der Wandung 73 und der Membran 77 wird erst nach Überschreiten eines sehr hohen Schwellendrucks Gas aus der Treibladungskartusche 7 abgegeben, wobei die Membran 77 bei Überschreiten dieses Schwellendrucks bricht. Die Verwendung einer langsam reagierenden Treibladung hat jedoch den Vorteil, dass der Gasdruck über längere Zeit konstant gehalten wird bzw. sich langsam abbaut, als im Fall eines schnell reagierenden Treibladungspulvers, wie dies in den Ausführungsformen nach den Fig. 1 bis 5 einsetzbar ist.

Denn bei den Ausführungsformen gemäß Fig. 1 bis 5 muss zur Erzeugung eines raschen Druckanstiegs im Bereich vor dem Kolben 9 infolge des Fehlens einer wesentlichen Verdämmung eine Treibladung verwendet werden, die zumindest eine schnell reagierende Komponente umfasst. Andernfalls würde der Treibspiegel 15 und damit der Kolben 9 bei diesen Ausführungsformen infolge eines langsamen Anstiegs des Drucks des Treibladungsgases relativ langsam beschleunigt.

Selbstverständlich kann jedoch auch bei den Ausführungsformen gemäß Fig. 1 bis 5 eine langsam reagierende Treibladung verwendet werden, wenn in analoger Weise eine Verdämmung verwendet wird, wie dies in Fig. 6 mittels der Wandung 73 und der Membran 77 erfolgt.

An dieser Stelle sei darauf hingewiesen, dass bei sämtlichen Ausführungsformen auch eine Treibladung eingesetzt werden kann, die eine langsam reagierende Komponente und eine schnell reagierende Komponente umfasst. Das Mischungsverhältnis und die Art der Verdämmung (falls erforderlich) ist so zu wählen, dass sich der erforderliche Verlauf des den Treibspiegel beaufschlagenden Gasdrucks ergibt.

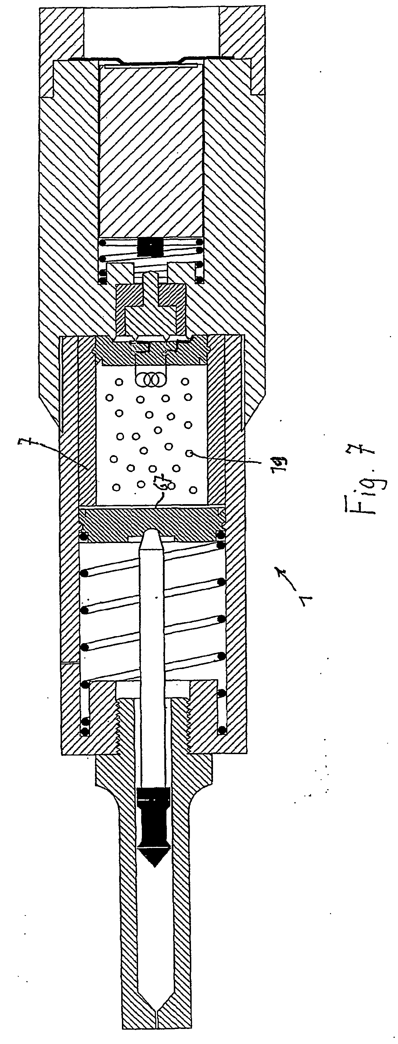

Die in Fig. 7 dargestellte Ausführungsform einer Injektionsvorrichtung 1 entspricht im Wesentlichen der Ausführungsform gemäß Fig. 6, wobei jedoch in diesem Fall wieder eine Treibladungskartusche 7 mit einer schnell reagierenden Treibladung 19 verwendet ist. Diese ist erforderlich, da die Treibladungskartusche 7 in ihrem vorderen Bereich nur mittels einer Membran 67 (analog der Membran der Treibladungskartusche in Fig. 5) verwendet ist. Eine wesentliche Verdämmungswirkung wird somit nicht erreicht.

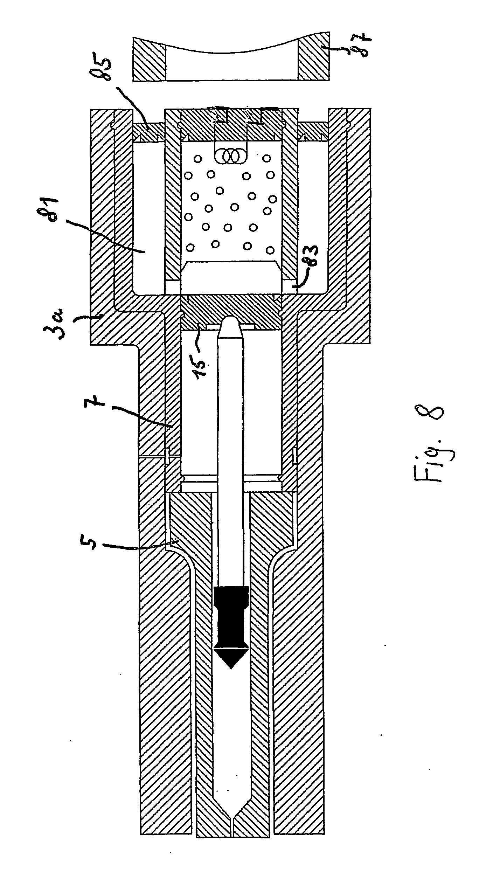

Fig. 8 zeigt eine weitere Ausführungsform einer Injektionsvorrichtung 1, die weitgehend der Ausführungsform in Fig. 1 entspricht. Die erfindungsgemäße Treibladungskartusche 7 ist jedoch zusätzlich mit einem Co-Volumen 81 versehen, welches den Raum, in welchem das Treibladungsgas erzeugt wird, ringförmig umgibt. Der ringförmige Raum des Co-Volumens 81 ist über Durchbrüche 83 mit dem Raum verbunden, in welchem das Treibgas erzeugt wird. Das Co-Volumen ermöglicht die Erzeugung eines höheren Treibgasvolumens, bis der Treibspiegel 15, der ebenfalls eine geringe Dämmwirkung erzeugt, aus seiner Ausgangsposition herausbewegt wird. Bei einer Vorwärtsbewegung des Treibspiegels 15 fällt der Druck des Treibladungsgases infolge des größeren erzeugten Volumens vor einer Bewegung des Treibspiegels langsamer ab.

Das Co-Volumen 81 ist durch ein ringförmiges Dichtelement 85 abgedichtet. Das ringförmige Dichtelement 85 kann mittels eines Verstellrings 87, der in Fig. 8 nur schematisch angedeutet ist, im Ringraum des Co-Volumens 81 verschoben werden. Der Verstellring 87 kann entweder fest mit dem rückwärtigen Gehäuseteil 3b verbunden sein, welches mit dem Gehäuseteil 3a verbindbar ist, oder als eigenständiges Teil ausgebildet sein, welches zwischen dem Dichtelement 85 und dem rückwärtigen Gehäuseteil 3b eingesetzt wird. Für unterschiedliche Einsatzzwecke, die unterschiedlich große Co-Volumina 81 erfordern, können verschiedene Verstellringe 87 mit unterschiedlichen axialen Ausdehnungen verwendet werden. Selbstverständlich ist es auch möglich, im Gehäuseteil 3b einen mittels einer Verstellmechanik axial verschiebbaren Verstellring 87 vorzusehen.

Zur Abdichtung des Dichtelements 85 kann dieses, wie vorstehend in Verbindung mit dem Treibspiegel beschrieben, an der Stirnseite, die vom Gasdruck beaufschlagt wird, zwei umlaufende Ausnehmungen aufweisen, die jeweils abdichtende lidartige Bereiche definieren.

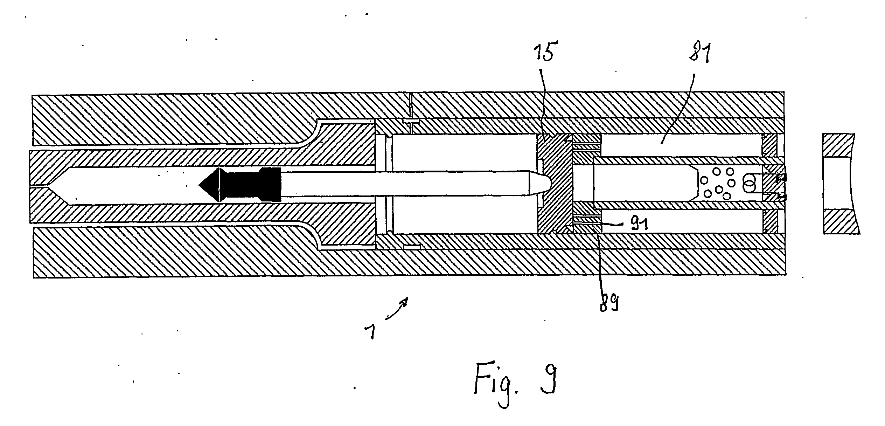

Auch die in Fig. 9 dargestellte Ausführungsform einer Injektionsvorrichtung 1 weist ein Co-Volumen 81 auf, welches sich ringförmig um den Raum erstreckt, in welchem das Treibladungsgas erzeugt wird. Zwischen dem Treibspiegel und dem ringförmigen Co-Volumen 81 ist bei dieser Ausführungsform eine Wandung 89 vorgesehen, in welcher mehrere Durchbrüche 91 vorgesehen sind.

Der Treibspiegel 15 beaufschlagt in seiner Ausgangsposition mit seiner rückseitigen Fläche die vordere stirnseitige Fläche der Wandung 89 und dichtet die Durchbrüche 81 ab. In der Ausgangsposition ist das Co-Volumen 81 daher nicht mit dem Raum verbunden, in welchem das Treibgas erzeugt wird. Erst nach dem Herausbewegen des Treibspiegels 15 aus seiner Ausgangsposition entsteht eine Verbindung zwischen dem Co-Volumen 81 und dem Volumen, in welchem das Treibgas erzeugt wird. Damit ergibt sich ein Verlauf des Gasdrucks, welcher den Treibspiegel beaufschlagt, der nach dem Herausbewegen des Treibspiegels 15 aus seiner Ausgangsposition von einem Peak auf einen geringeren Wert abfällt, diesen Wert jedoch länger aufrechterhält als dies ohne Co-Volumen der Fall wäre.

In den Fig. 10 und 11 ist nochmals dargestellt, mit welchem Treibladungskartuschentyp welcher Verlauf des Gasdrucks P realisiert werden kann, der den Treibspiegel beaufschlagt.

Fig. 10a zeigt eine rein gaserzeugende Treibladungskartusche 7, wie sie zuvor in Verbindung mit Fig. 6 beschrieben wurde. Durch die Verwendung einer relativ langsam reagierenden Treibladung (dargestellt durch grob angedeutete "Körner" oder "Tabletten" des Treibladungspulvers) und die Verdämmung mittels der Wandung 73 und der Membran 77 baut sich vor einem Durchbrechen der Membran 77 ein hoher Druck auf, der bei einem Herausbewegen des mit dem Treibgas beaufschlagten Treibspiegels aus seiner Ausgangsposition bei dem relativ geringen Kammervolumen der Treibladungskartusche 7 relativ schnell auf einen niedrigeren Wert abnimmt, wobei dieser Wert jedoch über längere Zeit gehalten werden kann, als dies mit einer ausschließlich schnell reagierenden Treibladung möglich wäre. Die verschiedenen in Fig. 10b dargestellten Verläufe des Drucks über die Zeit lassen sich mit unterschiedlichen Treibladungen realisieren. Die Kurve I kann z.B. mit einer noch relativ schnell reagierenden Treibladung erzeugt werden. Die Kurve II ist mittels einer langsamer reagierenden Treibladung realisierbar und die Kurve III mit einer sehr langsam reagierenden Treibladung oder der Kombination aus einer schnell reagierenden Treibladung und einer langsam reagierenden Treibladung.

Durch die Verdämmung der Ausführungsform der Treibladungskartusche 7 in Fig. 10a ergibt sich für alle drei Kurven I, II, III der selbe maximale Druck, bei welchem die Membran 77 durchbricht.

Bei der Ausführungsform einer rein gaserzeugenden Treibladungskartusche 7, wie sie zuvor auch in Verbindung mit der Ausführungsform einer Injektionsvorrichtung gemäß Fig. 7 beschrieben wurde, ist keine wesentlich verdämmende Wirkung gegeben. Die Membran 67 dient im Wesentlichen nur dazu, um die Treibladung 11 im Innenraum der Treibladungskartusche 7 zu halten. Dementsprechend ergibt sich bei einer langsam reagierenden Treibladung der Druckverlauf gemäß Kurve I in Fig. 11b. Die Membran 67 bricht bereits bei einem relativ geringen Maximaldruck, der dann auf einen niedrigeren Druck absinkt, der für eine relativ lange Zeit gehalten werden kann.

Bei einer schneller reagierenden Treibladung ergibt sich der Kurvenverlauf II in Fig. 11b. Die Membran 67 bricht wiederum bei einem Schwellendruck, der unterhalb des maximalen Drucks liegt. Der Maximaldruck ist im Wesentlichen nur vom Volumenstrom der Gaserzeugung abhängig. Dementsprechend ergibt sich ein höherer maximaler Druck als bei Kurve I, ein kürzerer zeitlicher Bereich, in dem der Druck annähernd konstant gehalten werden kann bzw. leicht abfällt und ein weiterer Bereich, in dem der Druck rasch auf Null abfällt.

Bei einer schnell reagierenden Treibladung ergibt sich ein sehr hoher maximaler Druck, praktisch kein Bereich, in dem der Druck über längere Zeit annähernd konstant gehalten werden kann, so dass die Kurve III relativ rasch vom Maximaldruck auf Null abfällt.

Abschließend sei darauf hingewiesen, dass selbstverständlich in Verbindung mit bestimmten Ausführungsformen beschriebene Merkmale der Injektionsvorrichtung, der Kanüle, der Treibladungskartusche und der Anzündeinrichtung auch analog auf andere Ausführungsformen übertragbar sind. Die dargestellten und/oder beschriebenen Ausführungsbeispiele der Treibladungskartuschen können gegebenenfalls auch für andere Verwendungszwecke eingesetzt werden, als für eine nadellose Injektionsvorrichtung.

## Patentansprüche

1. Treibladungskartusche für eine nadellose Injektionsvorrichtung,
a) mit einem Kartuschengehäuse (7), einer darin vorgesehenen aktivierbaren Treibladung (19) und einer Anzündvorrichtung (23) für das Aktivieren der Treibladung und
b) mit einem im Kartuschengehäuse angeordneten, gegenüber der Gehäuseinnenwandung abdichtenden Treibspiegel (15),
c) welcher durch den nach dem Anzünden der Treibladung (19) entstehenden Gasdruck zum Antreiben eines Kolbens (9) einer den zu injizierenden Wirkstoff enthaltenden Kanüle (5) aus einer Ausgangsposition in eine Endposition bewegbar ist und dessen Außenumfang gegenüber dem Innenumfang des Kartuschengehäuses auch während der gesamten Verschiebebewegung abdichtend ausgebildet ist,
**dadurch gekennzeichnet,**
d) dass das jeweilige Gehäuse ein Co-Volumen (81) aufweist, welches mit dem Volumen des Gehäuses, in welchem das Gas erzeugbar ist, in der Ausgangsposition des Treibspiegels (15) verbunden oder durch das Bewegen des Treibspiegels (15) aus der Ausgangsposition heraus verbindbar ist.

2. Kanülen-/Treibladungskartuscheneinheit für eine Vorrichtung zur nadellosen Injektion,
a) mit einem Kanülen-/Kartuschengehäuse, einer darin vorgesehenen aktivierbaren Treibladung (19) und einer Anzündvorrichtung (23) für das Aktivieren der Treibladung,
b) mit einem im Kanülen-/Kartuschengehäuse abdichtend verschiebbaren Kolben (9) für das Auspressen des Wirkstoffs (11) aus einer im Kanülengehäuse vorgesehenen Austrittsöffnung (53) und
c) mit einem im Kanülen-/Kartuschengehäuse angeordneten, gegenüber der Gehäuseinnenwandung abdichtenden Treibspiegel (15), welcher durch den nach dem Anzünden der Treibladung (19) entstehenden Gasdruck zum. Antreiben des Kolbens (9) aus einer Ausgangsposition in eine Endposition bewegbar ist und dessen Außenumfang gegenüber dem Innenumfang des Kanülen-/Kartuschengehäuses auch während der gesamten Verschiebebewegung abdichtend ausgebildet ist,
**dadurch gekennzeichnet,**
d) dass das jeweilige Gehäuse ein Co-Volumen (81) aufweist, welches mit dem Volumen des Gehäuses, in welchem das Gas erzeugbar ist, in der Ausgangsposition des Treibspiegels (15) verbunden oder durch das Bewegen des Treibspiegels (15) aus der Ausgangsposition heraus verbindbar ist.

3. Treibladunskartusche oder Kanülen-/Treibladungskartuscheneinheit nach Anspruch 1 oder 2, **dadurch gekennzeichnet, dass** das Co-Volumen (81) als Ringraum ausgebildet ist, der sich um das Volumen erstreckt, in welchem das Gas erzeugbar ist.

4. Treibladungskartusche oder Kanülen-/Treibladungskartuscheneinheit nach Anspruch 3, **dadurch gekennzeichnet, dass** der Ringraum durch eine stirnseitige Wandung (89) begrenzt ist, in welcher ein oder mehrere Durchbrüche (91) vorgesehen sind, die in der Ausgangsposition durch den Treibspiegel (15) im Wesentlichen abgedichtet sind, dass in der stirnseitigen Wandung (89) wenigstens ein Durchbruch vorgesehen ist, durch welchen das durch die Treibladung erzeugte Gas den Treibspiegel beaufschlagt.

5. Treibladungskartusche oder Kanülen-/Treibladungskartuscheneinheit nach Anspruch 3 oder 4, **dadurch gekennzeichnet, dass** das Co-Volumen (81) des Ringraums durch ein an der rückwärtigen Stirnseite des Ringraums eingreifendes ringförmiges Element einstellbar ist.

## Claims

1. Propellant charge cartridge for a needle-less injection device,
a) with a cartridge housing (7), an activatable propellant charge (19) provided therein, and an ignition device (23) for the activation of the propellant charge, and
b) with a sabot (15) which is arranged inside the cartridge housing and seals with respect to the inner wall of the housing,
c) the sabot (15) can be moved from a starting position to an end position by the gas pressure that is generated after the ignition of the propellant charge (19) and serves to propel a plunger (9) of a cannula (5) containing the agent to be injected, and the outer circumference of the sabot (15) is provided to provide a seal with respect to the inner circumference of the cartridge housing, including during the entire displacement motion,
**characterised in that**
d) the respective housing comprises a co-volume (81) which communicates with the volume of the housing, in which the gas can be generated, when the sabot (15) is in its starting position or can be made to communicate by moving the sabot (15) out of its starting position.

2. Cannula/propellant charge cartridge unit for a device for needle-less injection,
a) with a cannula/cartridge housing, an activatable propellant charge (19) provided therein, and an ignition device (23) for the activation of the propellant charge,
b) with a plunger (9), which is displaceable while providing a seal inside the cannula/cartridge housing and serves to expel the agent (11) from an exit opening (53) provided in the cannula housing, and
c) with a sabot (15) which is arranged inside the cannula/cartridge housing and seals with respect to the inner wall of the housing and which can be moved from a starting position to an end position by the gas pressure, which is generated after the ignition of the propellant charge (19) and serves to propel the plunger (9), and the outer circumference of the sabot (15) is provided to provide a seal with respect to the inner circumference of the cannula/cartridge housing, including during the entire displacement motion,
**characterised in that**
d) the respective housing comprises a co-volume (81) which communicates with the volume of the housing, in which the gas can be generated, when the sabot (15) is in its starting position or can be made to communicate by moving the sabot (15) out of its starting position.

3. Propellant charge cartridge or cannula/propellant charge cartridge unit according to claim 1 or 2, **characterised in that** the co-volume (81) is provided in the form of an annular space extending around the volume, in which the gas can be generated.

4. Propellant charge cartridge or cannula/propellant charge cartridge unit according to claim 3, **characterised in that** the annular space is limited by a front side wall (89), in which one or multiple breakthroughs (91) are provided, which are essentially sealed by the sabot (15) in its starting position, and **in that** there is at least one breakthrough provided in the front side wall (89), through which the gas generated by the propellant charge applies pressure to the sabot.

5. Propellant charge cartridge or cannula/propellant charge cartridge unit according to claim 3 or 4, **characterised in that** the co-volume (81) of the annular space is adjustable by means of a ring-shaped element engaging the rear front side of the annular space.

## Revendications

1. Cartouche de charge propulsive pour un dispositif d'injection sans aiguille,
a) avec un boîtier de cartouche (7), une charge propulsive (19) activable prévue dedans et un dispositif d'allumage (23) pour activer la charge propulsive et
b) avec un miroir propulseur (15) disposé dans le boîtier de cartouche, assurant l'étanchéité par rapport à la paroi intérieure du boîtier,
(c) qui est déplaçable d'une position initiale dans une position finale par la pression de gaz se produisant après l'allumage de la charge propulsive (19) pour entraîner un piston (9) d'une canule (5) contenant la substance active à injecter et dont le pourtour extérieur est conçu pour assurer l'étanchéité par rapport au pourtour intérieur du boîtier de cartouche également pendant tout le mouvement de déplacement,
**caractérisée en ce que**
d) le boîtier respectif comprend un volume de Co (81) qui est relié au volume du boîtier dans lequel le gaz doit être produit dans la position initiale du miroir propulseur (15) ou qui peut être relié par le mouvement du miroir propulseur (15) hors de la position initiale.

2. Ensemble canule/cartouche de charge propulsive pour un dispositif d'injection sans aiguille,
a) avec un boîtier de canule/cartouche, une charge propulsive (19) activable prévue dedans et un dispositif d'allumage (23) pour activer la charge propulsive,
b) avec un piston (9) déplaçable de manière assurant l'étanchéité dans le boîtier de canule/cartouche pour extraire par pression la substance active (11) hors d'un orifice de sortie (53) prévu dans le boîtier de la canule et
c) avec un miroir propulseur (15) disposé dans le boîtier de canule/cartouche, assurant l'étanchéité par rapport à la paroi intérieure du boîtier qui est déplaçable d'une position initiale dans une position finale par la pression de gaz se produisant après l'allumage de la charge propulsive (19) pour entraîner le piston (9) et dont le pourtour extérieur est conçu pour assurer l'étanchéité par rapport au pourtour intérieur du boîtier de canule/cartouche également pendant tout le mouvement de déplacement,
**caractérisé en ce que**
d) le boîtier respectif comprend un volume de Co (81) qui est relié au volume du boîtier dans lequel le gaz doit être produit dans la position initiale du miroir propulseur (15) ou qui peut être relié par le mouvement du miroir propulseur (15) hors de la position initiale.

3. Cartouche de charge propulsive ou ensemble canule-cartouche de charge propulsive selon la revendication 1 ou 2, **caractérisée en ce que** le volume de Co (81) est conçu comme un espace annulaire qui s'étend autour du volume dans lequel le gaz doit être produit.

4. Cartouche de charge propulsive ou ensemble canule-cartouche de charge propulsive selon la revendication 3, **caractérisée en ce que** l'espace annulaire est limité par une paroi frontale (89) dans laquelle sont prévus un ou plusieurs percements (91) qui sont essentiellement étanchés par le miroir propulseur (15) dans la position initiale, **en ce qu'**au moins un percement est prévu dans la paroi frontale (89) à travers lequel le gaz généré par la charge propulsive alimente le miroir propulseur.

5. Cartouche de charge propulsive ou ensemble canule-cartouche de charge propulsive selon la revendication 3 ou 4, **caractérisée en ce que** le volume de Co (81) de l'espace annulaire est réglable au moyen d'un élément annulaire venant en prise sur la face arrière de l'espace annulaire.
